Europäisches Patentamt

European Patent Office

Office européen des brevets

(11) Publication number: **0 380 184**
**A1**

# (12) EUROPEAN PATENT APPLICATION

(21) Application number: **90200174.2**

(22) Date of filing: **23.01.90**

(51) Int. Cl.⁵: **C10K 1/34**

(30) Priority: **25.01.89 GB 8901577**

(43) Date of publication of application:
**01.08.90 Bulletin 90/31**

(84) Designated Contracting States:
**BE DE ES GB IT NL SE**

(71) Applicant: **SHELL INTERNATIONALE RESEARCH MAATSCHAPPIJ B.V.**
**Carel van Bylandtlaan 30**
**NL-2596 HR Den Haag(NL)**

(72) Inventor: **Wang, Henry Kwok Hing**
**Carel van Bylandtlaan 30**
**NL-2596 HR Den Haag(NL)**

(74) Representative: **Aalbers, Onno et al**
**P.O. Box 302**
**NL-2501 CH The Hague(NL)**

(54) **Removing hydrogen cyanide and carbon oxysulphide from a gas mixture.**

(57) Process for removing hydrogen cyanide and carbon oxysulphide from a gas mixture comprising contacting the gas mixture in the presence of water with a first catalyst (5) comprising molybdenum on an alumina carrier and with a second catalyst (9) comprising a Group VIb metal and an alkali metal at a temperature of between 150 and 200 °C and a gas hourly space velocity of between 4 000 and 6 000 Nm³ gas mixture per m³ catalyst per hour.

EP 0 380 184 A1

## REMOVING HYDROGEN CYANIDE AND CARBON OXYSULPHIDE FROM A GAS MIXTURE

The present invention relates to removing hydrogen cyanide and carbon oxysulphide from a gas mixture, wherein these components are hydrolysed according to the following reaction equations:

$HCN + H_2O <===> NH_3 + CO$

$HCN + 2H_2O <===> CO_2 + NH_3 + H_2$

$COS + H_2O <===> H_2S + CO_2$.

An example of a gas mixture containing hydrogen cyanide and carbon oxysulphide is gas obtained from partial combusting carbonaceous material, such as coal, or hydrocarbon-containing material. Such a gas mixture will in general comprise between 0.50 and 0.60 mol carbon monoxide/mol, between 0.20 and 0.30 mol hydrogen/mol, nitrogen, carbon dioxide, hydrogen sulphide, carbon oxysulphide, hydrogen cyanide, ammonia, methane and water, the amount of carbon oxysulphide is between 180 and 530 ppmv (volume parts per million) and the amount of hydrogen cyanide is between 140 and 190 ppmv.

Two further reactions which can take place are the so-called water-gas shift reaction $CO + H_2O <===> CO_2 + H_2$ and the sour-gas shift reaction $CO + H_2S <===> COS + H_2$. Both the water-gas shift reaction and the sour-gas shift reaction are exothermic reactions.

It will be appreciated that these reactions compete with the hydrolysis reactions. Therefore it is an object of the present invention to provide a process for removing hydrogen cyanide and carbon oxysulphide from a gas mixture in which the water-gas shift reaction and the sour-gas shift reaction are suppressed.

To this end the process according to the invention comprises contacting the gas mixture in the presence of water with a first catalyst comprising molybdenum on an alumina carrier and with a second catalyst comprising a Group VIb metal and an alkali metal at a temperature of between 150 and 200 $^\circ$C and a gas hourly space velocity of between 4 000 and 6 000 $Nm^3$ gas mixture per $m^3$ catalyst per hour.

Suitably the gas mixture is first contacted with the first catalyst and subsequently with the second catalyst.

In the specification and in the claim reference is made to the gas hourly space velocity. This is the gas hourly space velocity for each of the two catalysts. The gas volume is expressed in $Nm^3$, 1 $Nm^3$ gas being 1 $m^3$ gas at standard conditions of temperature and pressure.

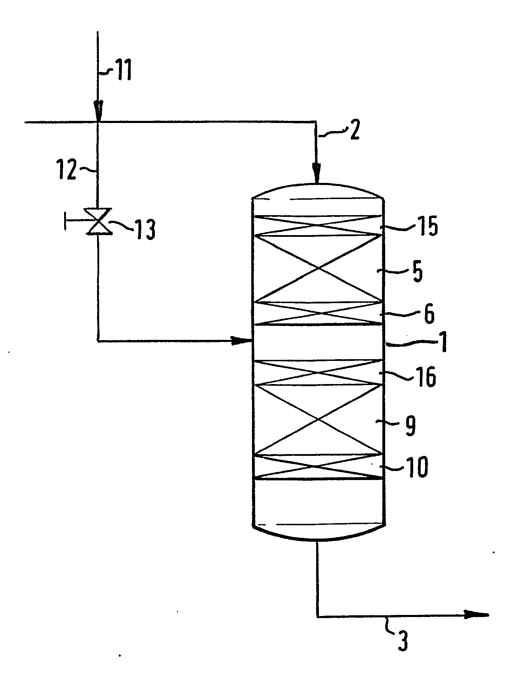

The invention will now be described by way of example in more detail with reference to the accompanying drawing showing schematically a partial cross-section of the apparatus used in carrying out the process according to the invention. The apparatus comprises a reactor 1 provided with a supply conduit 2 and outlet conduit 3. In the reactor 1 there are provided two catalyst beds namely a first catalyst bed 5 which is arranged on catalyst support 6 and which consists of a first catalyst comprising molybdenum on an alumina carrier, and a second catalyst bed 9 which is arranged on catalyst support 10 and which consists of a second catalyst comprising a Group VIb metal and an alkali metal on an alumina carrier.

During normal operation $168*10^3$ $Nm^3$ gas mixture per hour is supplied through the conduit 2 to the reactor 1 at a temperature of 180 $^\circ$C and a pressure of 23 bar absolute. The composition of the gas mixture is as follows: 0.25885 mol $H_2$/mol, 0.05805 mol $N_2$/mol, 0.59288 mol CO/mol, 0.0144 mol $CO_2$/mol, 0.00153 mol $H_2S$/mol, 0.00014 mol HCN/mol (= 140 ppmv HCN), 0.00018 mol COS/mol (= 180 ppmv COS), 0.00017 mol $NH_3$/mol, 20.0001 mol $CH_4$/mol and 0.07371 mol $H_2O$/mol. In the reactor 1 the gas mixture is first contacted with 33.7 $m^3$ of first catalyst arranged in first catalyst bed 5 and subsequently with 33.7 $m^3$ of second catalyst arranged in second catalyst bed 9. The gas hourly space velocity for the first catalyst is $168*10^3/33.7 = 5*10^3$ $Nm^3$ gas per $m^3$ of catalyst per hour, and the gas hourly space velocity for the second catalyst bed is equal thereto. The first catalyst consists of 4% by mass of molybdenum on an alumina carrier, and the second catalyst is Girdler G-41-P (trade name) which comprises 10% by mass of chromium dioxide on alumina and promoted with potassium.

Treated gas is removed from the reactor 1 through conduit 3 and is supplied to a plant (not shown) for removing hydrogen sulphide from the gas mixture. The treated gas contains only 10 ppmv hydrogen cyanide and 20 ppmv carbon oxysulphide. In addition the amount of carbon dioxide in the treated gas is substantially the same as the amount of carbon dioxide in the gas mixture as supplied to the reactor 1.

It is found that with the process according to the invention a good conversion can be obtained and the water-gas shift reaction and the sour-gas shift reaction are suppressed.

Suitably the the gas mixture contains between 0.02 and 0.10 mol water per mol gas mixture.

If the water content of the gas mixture is too low, water can be added to the gas mixture via conduit 11.

Furthermore, part of the gas mixture can be supplied directly to the second catalyst through conduit 12 provided with valve 13. This is done to add additional water in the second catalyst bed, and furthermore, to control the temperature in the lower part of the reactor.

The gas mixture may contain materials such as nickel, cobalt, iron or copper which can promote the water-gas shift reaction and the sour-gas shift reaction. These material are present in the gas mixture as fly ash. To remove these materials from the gas mixture the reactor is provided with a plurality of adsorbent layers 15 and 16 of inert adsorbent material, such as ceramic balls. The volume of the layers is so small (about 1/3rd of the volume of a catalyst bed) that the velocity of the gas mixture passing through adsorbent layers 15 and 16 is sufficiently high and consequently the residence time of the gas is sufficiently short to minimize the extent that the water-gas shift reaction and the sour-gas shift reaction can progress.

## Claims

1. Process for removing hydrogen cyanide and carbon oxysulphide from a gas mixture comprising contacting the gas mixture in the presence of water with a first catalyst comprising molybdenum on an alumina carrier and with a second catalyst comprising a Group VIb metal and an alkali metal at a temperature of between 150 and 200 $^\circ$C and a gas hourly space velocity of between 4 000 and 6 000 $Nm^3$ gas mixture per $m^3$ catalyst per hour.

2. Process as claimed in claim 1, wherein the gas mixture is first contacted with the first catalyst and subsequently with the second catalyst.

3. Process as claimed in claim 1 or 2, wherein the second catalyst comprises chromium and potassium.

4. Process as claimed in claim 2 or 3, wherein part of the gas mixture is supplied directly to the second catalyst.

5. Process as claimed in any one of the claims 1-4, wherein the the gas mixture contains between 0.02 and 0.10 mol water per mol gas mixture.

6. Process for removing hydrogen cyanide and carbon oxysulphide from a gas mixture substantially as described in the specification with reference to the accompanying drawing.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | FR-A-1541573 (SHELL INTERNATIONALE RESEARCH MAATSCHAPPIJ N.V.) * the whole document * | 1 | C10K1/34 |
| A | FR-A-2512052 (KRUPP-KOPPERS GMBH) * claims 1-4 * | 1 | |
| A,P | EP-A-324526 (COMPRIMO B.V.) * claims 1, 2, 8, 11 * | 1, 2, 5 | |

TECHNICAL FIELDS
SEARCHED (Int. Cl.5 )

C10K
B01D

The present search report has been drawn up for all claims

| Place of search | Date of completion of the search | Examiner |
|---|---|---|
| BERLIN | 05 APRIL 1990 | BERTRAM H.E.H. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P0401)